# EUROPEAN PATENT SPECIFICATION

(11) **EP 0 985 652 B1**
(45) Date of publication and mention of the grant of the patent: **17.04.2002**
(21) Application number: 99123813.0
(22) Date of filing: 07.12.1996
(51) Int. Cl.: C07C 49/675, C07C 45/36

(54) **Method for production of fluorenone**
Verfahren zur Herstellung von Fluorenon
Procédé pour la préparation de fluorénone

(30) Priority: 11.12.1995 JP 32137095; 11.12.1995 JP 32137195
(43) Date of publication of application: 15.03.2000
(62) Divisional of application: 96119656.5
(73) Proprietor: NIPPON SHOKUBAI CO., LTD., Chuo-ku, Osaka-shi, Osaka-fu 541 (JP)
(72) Inventor: Takahashi, Tsukasa, Himeji-shi, Hyogo-ken (JP); Emoto, Yasuhisa, Ibo-gun, Hyogo-ken (JP)
(74) Representative: Luderschmidt, Schüler & Partner GbR

(56) References cited:
- PATENT ABSTRACTS OF JAPAN vol. 010, no. 103 (C-340), 18 April 1986 (1986-04-18) & JP 60 233028 A (NIPPON SHOKUBAI KAGAKU KOGYO KK), 19 November 1985 (1985-11-19)

## Description

This invention relates to a method for the production of fluorenone and more particularly to a method for producing fluorenone by the vapor-phase catalytic oxidation of fluorene with a high yield. Fluorenone is a commercially useful substance as raw materials for agricultural pesticides, medicines, and functional macromolecular compounds.

### Description of the Prior Art:

The method for producing fluorenone by the vapor-phase catalytic oxidation of fluorene with molecular oxygen is at an advantage in enjoying high productivity as compared with the liquid-phase method and avoiding emission of waste liquor. It is nevertheless at a disadvantage in having to sacrifice selectivity when the reaction is performed at a high conversion. It incurs difficulty in effectively utilizing fluorene while maintaining high productivity. It does not easily produce fluorenone stably with high yield because it entails the phenomenon of suffering the selectivity of fluorenone to be lowered and the catalytic activity to be varied, depending on the quality of the raw material fluorene to be used.

The method for the production of fluorenone is known in two types, the liquid-phase method and the vapor-phase method. As to the liquid-phase method, many reports have been published on the method of liquid-phase oxidation with molecular oxygen by the use of a phase-transfer catalyst formed of an aqueous alkali solution, a hydrophobic organic solvent, and a quaternary ammonium salt (JP-A-07-82,206 and JP-A-07-82,207). As to the vapor-phase method, a method using vanadium pentoxide (U.S. Patent No. 1,374,695), a method using a catalyst formed of iron vanadate and potassium sulfate [Zh. Pyirl Khim 35, 693-696 (1962)], a method using a catalyst formed of vanadium pentoxide and tin oxide [Engineering Chemical Journal, 56, (6), 413-416 (1953)], a method for causing inclusion of a large volume of water by the use of a catalyst formed of metal salts and alkali metal sulfates of vanadic acid, molybdic acid, or tungstic acid (U.S. Patent No. 1,892,768), a method using a catalyst formed of vanadium pentoxide, silica, and potassium sulfate (U.S. Patent No. 2,956,065), and a method using a catalyst formed of vanadium, titania, and an alkali metal (JP-A-60-233,028) have been disclosed. In addition to these methods, a method using a catalyst formed of vanadium, iron, and cesium [Stud, Surf, Sci. Catal. (1993), 75 (New Frontiers in Catalysis, Pt. A) , 707-17] has been also disclosed.

It has been heretofore known that a vanadium-based catalyst can be used as an effective catalyst for the production of fluorenone by the vapor-phase oxidation of fluorene. It has been difficult, however, to obtain fluorenone stably with a high yield because the reaction performed at a high conversion results in lowering the selectivity and the quality of the raw material lowers the selectivity or changes the catalytic activity.

An object of this invention, therefore, is to provide an improved method for the production of fluorenone.

Another object of this invention is to solve the problems heretofore attendant on the vapor-phase oxidation and provide a method for producing fluorenone stably with high selectivity.

### SUMMARY OF THE INVENTION

The objects mentioned above are accomplished, in the production of fluorenone by the vapor-phase catalytic oxidation of fluorene with a molecular oxygen-containing gas, by a method for the production of fluorenone which comprises keeping the sulfur concentration in the raw material fluorene at or below 0.15% by weight.

Our study has evolved a knowledge that in the vapor-phase catalytic oxidation of fluorene with a molecular oxygen-containing gas, the fluorenone is obtained at a high conversion with high selectivity by adjusting the fluorene concentration in the feed raw material gas consisting of fluorene as a raw material and the molecular oxygen-containing gas within a range higher than that of a conventional method. This invention has been perfected as a result of this knowledge. We have tested various species of fluorene as the raw material and studied about the conditions for stably producing fluorenone by the vapor-phase catalytic oxidation, to find that the sulfur component in the raw material fluorene affects the catalytic activity and the selectivity of fluorenone. We have perfected this invention based on this knowledge.

According to this invention, fluorenone can be produced at a high conversion with high selectivity by the vapor-phase catalytic oxidation of fluorene. Particularly, this invention accomplishes the high selectivity with the conversion of fluorene kept at such a high level as that approximating closely to 100%.

### BRIEF DESCRIPTION OF THE DRAWINGS

Fig. 1 is a schematic diagram of a reaction apparatus to be used in working examples and controls herein.

### DESCRIPTION OF THE PREFERRED EMBODIMENT

The fluorene to be used as the raw material in this invention may be any of various species of fluorene such as, for example, the fluorene which is obtained from a fluorene-containing oil derived from coal tar (tar type fluorene) and the fluorene which is obtained from the residual oil by-produced in the process for the production of dealkylated benzene (petroleum process type fluorene). The raw material fluorene does not need to completely consist of fluorene but may contain various impurities arising from the starting raw material or the process of purification such as, for example, biphenyl compounds like methyl biphenyl, naphthalene compounds like methyl naphthalene, fluorene derivatives like 9-methyl fluorene, and dibenzofuran.

Even when the fluorene concentration in the raw material fluorene is not more than 60% by weight, the production of fluorenone may be obtained with a high yield. In consideration of the necessity for a subsequent step for purification, however, it is appropriate to use the raw material fluorene having a fluorene concentration of not less than 70% by weight, preferably not less than 80% by weight.

The catalyst to be used for the vapor-phase catalytic reaction according to this invention is not particularly limited. Any of the catalysts that are generally used for the vapor-phase oxidation of fluorene into fluorenone can be used. As concrete examples of the catalyst which is effectively usable herein, a catalyst formed of vanadium pentoxide, a catalyst formed of iron vanadate and potassium sulfate, a catalyst formed of vanadium pentoxide and tin oxide, a catalyst formed of a metal salt or an alkali metal sulfate of vanadic acid, molybdic acid, or tungstic acid, a catalyst formed of vanadium pentoxide, silica, and potassium sulfate, a catalyst formed of vanadium, titania, and an alkali metal, and a catalyst formed of vanadium, iron, and cesium sulfate may be cited. Among other catalysts mentioned above, the catalyst formed of vanadium, titanium oxide, and an alkali metal sulfate is used particularly advantageously. Here, the atomic ratio of alkali metal element/vanadium appropriately falls in the range of 0.8 to 8, preferably 1 to 5, particularly preferably 1 to 3. It is particularly advantageous to use a supported catalyst having such a catalytically active component as mentioned above deposited on such an inert carrier as of silicon carbide, alumina, silica, silica-alumina, or pumice. Incidentally, the carried catalyst is in the form of spheres, cylinders, saddles, or cylindricalparticles which generally have a particle diameter in the range of 3 to 15 mm, preferably 3 to 10 mm.

Characteristic of this invention resides in keeping the sulfur concentration in the raw material fluorene at or below 0.15% by weight, preferably at or below 0.1% by weight, and more preferably at or below 0.05% by weight. If the sulfur concentration in the raw material fluorene exceeds 0.15% by weight and reaches 0.2% by weight, for example, the production of fluorenone will not be attained with high yield (refer to below Controls) . The reason for this critical sulfur concentration remains yet to be elucidated. It may be logically explained, however, by a supposition that the sulfur heightens the activity of the oxidizing catalyst used in the reaction and abnormally promotes the oxidizing reaction or the combustion reaction. When the raw material fluorene having a sulfur concentration of 0.2% by weight is used for the oxidizing reaction, for example, the temperature of the hot spots in the catalyst bed rises to encourage the combustion reaction and notably lower the selectivity of fluorenone. When the sulfur concentration in the raw material fluorene is decreased substantially to 0, the rise of the temperature of the hot spots can be effectively repressed and the reaction can be stably continued.

The term "sulfur concentration" as used in this invention refers to the concentration as sulfur of the total amount of organic and inorganic sulfur compounds present in the raw material fluorene, as determined by "the method for testing sulfur content of petroleum product by means of combustion tube" specified in JIS (Japanese Industrial Standard) K-2547.

The sulfur concentration in the raw material fluorene can be adjusted at or below 0.15% by weight by purification due to distillation or crystallization or by various methods used for the desulfurization of petroleum. The method to be used for this purpose is not particularly limited. It is particularly proper that the sulfur concentration should be not more than 0.05% by weight.

The reaction conditions such as the temperature, the spatial velocity, and the like to be involved in the vapor-phase catalytic oxidation contemplated by this invention are not limited particularly but may be suitably selected, depending on the kind of the catalyst to be used, for example.

Generally, the reaction temperature is selected in the range of 250° to 480 ° C, preferably 300° to 450 ° C and the spatial velocity in the range of 100 to 10,000/hr, preferably 200 to 5,000/hr.

Now, this invention will be described more specifically below with reference to working examples.

### Preparation Example 1

A homogenous solution was obtained by stirring 13.4 g of ammonium vanadate, 26.8 g of oxalic acid, 2.4 g of potassium sulfate, and 25.1 g of cesium sulfate in 180 ml of pure water at 80°C. This solution was cooled to room temperature. A homogeneous slurry was formed by thoroughly stirring 120 g of anatase type titanium dioxide having a surface area of 20 m²/g and 4.8 g of silicon carbide whiskers in the cooled solution. A slurry for the preparation of catalyst was obtained by adding 400 ml of pure water to the resultant slurry.

A stainless steel heatable rotary drum was charged with 200 g of beads of silicon carbide carrier of 4 mm in average diameter, and set rotating. The silicon carbide carrier in the rotating kiln was kept at a temperature in the range of 180° to 220 °C and 20 g of the slurry for the preparation of catalyst mentioned above was sprayed and deposited on the beads of silicon carbide carrier. The beads now having the catalyst carded thereon were calcined as swept with a stream of air at 550 ° C for five hours, to prepare an oxidizing catalyst. The atomic ratio of each added element was as follows.
V : Cs : K : S : TiO₂ = 7.64 : 9.26 : 1.84 : 5.55 : 100

### Control 1

In the same apparatus as illustrated in Fig. 1, the oxidizing catalyst mentioned above was packed in a bed length of 250 mm on the gas outlet side of the stainless steel reaction tube of 25 mm in inside diameter. This reaction tube was retained in a molten salt bath at 430 °C. A raw material fluorene of the tar type having the following composition and supplied from a fluorene raw material feeder via a pipe kept at 140 °C was supplied via the pipe immersed in the molten salt bath so as to be mixed with preheated air and introduced into the catalyst bed.

### Composition of raw material (% by weight)

Fluorene (75)
Sulfur (0.2)
9-Methyl fluorene, methyl biphenyl, dibenzofuran, etc. (balance)

The reaction of fluorene and air was carried out with the feed rate of fluorene fixed at 39.3mg/minute (as pure fluorene) and the air flow fixed at 2050 ml/minute (0 °C under one atmosphere).

The gas produced by the reaction was collected via an air-cooled glass collecting tube of 35 mm in inside diameter, and three aerating vials arranged in series and filled with acetone. It was recovered in the form of an acetone solution and then analyzed with a gas chromatography using a column OV-1/0.25 mm ID/50 m (produced by Shimadzu Seisakusho, Ltd. and marketed under product code of "GC-14B"). Consequently, the conversion of fluorene was found to be 94.2 mol%, the selectivity of fluorenone to be 78.7 mol%, and the yield of fluorenone to be 74.1 mol%.

### Example 1

The reaction was performed by following the procedure of Control 1 while using a raw material fluorene originating in the petroleum process containing no sulfur and having the following composition instead. The results are shown in Table 1.

### Composition of raw material (% by weight)

Fluorene (96)
Sulfur (0)
Methyl biphenyl, methyl naphthalene, 9-methyl fluorene, etc. (balance)

### Example 2

The reaction was performed by following the procedure of Control 1 while using a raw material fluorene originating in the tar and having the following composition instead. The results are shown in Table 1.

### Composition of raw material (% by weight)

Fluorene (94)
Sulfur (0.05)
9-Methyl fluorene, methyl biphenyl, dibenzofuran, etc. (balance)

### Example 3

The reaction was performed by following the procedure of Control 1 while using a raw material fluorene originating in the petroleum process containing no sulfur and having the following composition instead. The results are shown in Table 1.

### Composition of raw material (% by weight)

Fluorene (94)
Sulfur (0)
Methyl biphenyl, methyl naphthalene, 9-methyl fluorene, etc. (balance)

**Table 1**

| | Sulfur content (% by weight) | Conversion of fluorene (mol%) | Selectivity of fluorenone (mol%) | Yield of fluorenone (mol%) |
|---|---|---|---|---|
| Control 1 | 0.2 | 94.2 | 78.7 | 74.1 |
| Example 1 | 0 | 90.5 | 90.2 | 81.6 |
| Example 2 | 0.05 | 89.7 | 89.1 | 80.0 |
| Example 3 | 0 | 89.4 | 92.1 | 82.3 |

### Example 4

The reaction was carried out by following the procedure of Example 1 while changing the gas concentration to 460 g/Nm³. The conversion of fluorene was found to be 93.1 mol%, the selectivity of fluorenone to be 95.8 mol%, and the yield of fluorenone to be 89.2 mol%. The highest temperature of the catalyst bed was 480° C.

### Control 2

The reaction was carried out by following the procedure of Example 4 while using the raw material fluorene used in Control 1 instead. The highest temperature of the catalyst bed surpassed 560 °C. Since the temperature still continued to rise, the reaction was terminated.

## Claims

1. A method for the production of fluorenone by the vapor-phase catalytic oxidation of fluorene with a molecular oxygen-containing gas, **characterized in** keeping the sulfur content in the raw material fluorene at or below 0.15% by weight.

2. A method according to claim 1, wherein said molecular oxygen-containing gas is air.

3. A method according to claim 1, wherein said catalyst contains at least vanadium, titania, and an alkali metal and the atomic ratio of said alkali metal to vanadium is in the range of 0.8 to 8.

## Patentansprüche

1. Verfahren zur Herstellung von Fluorenon durch katalytische Dampfphasenoxidation von Fluoren mit einem molekularen sauerstoffhaltigem Gas, **dadurch gekennzeichnet, dass** man den Schwefelgehalt in dem Fluoren-Ausgangsmaterial bei oder unterhalb von 0,15 Gew.% hält.

2. Verfahren gemäß Anspruch 1, bei dem das molekulare sauerstoffhaltige Gas Luft ist.

3. Verfahren gemäß Anspruch 1, bei dem der Katalysator mindestens Vanadium, Titandioxid und ein Alkalimetall enthält, und das Atomverhältnis des Alkalimetalls zum Vanadium im Bereich von 0,8 bis 8 liegt.

## Revendications

1. Procédé pour la préparation de fluorénone par l'oxidation catalytique de fluorène en phase vapeur avec un gaz contenant d'oxygène moléculaire, **caractérisé en ce que** le teneur en soufre dans la matière première fluorène est maintenu à 0,15% en poids ou à moins.

2. Procédé d'après la revendication 1, dans lequel ledit gaz contenant d'oxygène moléculaire est de l'air.

3. Procédé d'après la revendication 1, dans lequel ledit catalyseur comporte au moins le vanadium, l'oxyde titanique et un métal alcalin et que la proportion atomique dudit métal alcalin par rapport au vanadium est dans la fourchette de 0,8 à 8.
